# EUROPEAN PATENT SPECIFICATION

(11) **EP 3 317 254 B1**
(45) Date of publication and mention of the grant of the patent: **03.10.2018**
(21) Application number: 16795045.0
(22) Date of filing: 14.11.2016
(51) Int. Cl.: C07D 231/20

(54) **METHOD FOR PREPARATION OF 1-METHYL-3-(TRIFLUOROMETHYL)-1HPYRAZOL-5-OL**
VERFAHREN ZUR HERSTELLUNG VON 1-METHYL-3-(TRIFLUORMETHYL)-1H-PYRAZOL-5-OL
PROCÉDÉ DE PRÉPARATION DE 1-MÉTHYL-3- (TRIFLUOROMÉTHYL) -1H-PYRAZOL-5-OL

(30) Priority: 16.11.2015 US 201562255775 P; 16.11.2015 EP 15194830; 18.11.2015 EP 15195144; 19.02.2016 EP 16156409; 19.04.2016 EP 16165975; 04.07.2016 EP 16177676
(43) Date of publication of application: 09.05.2018
(73) Proprietor: Lonza Ltd, 3930 Visp (CH)
(72) Inventor: TAESCHLER, Christoph, 3930 Visp (CH); GIRARD, Christophe, 1950 Sion (CH); TONAZZI, Sandro, 8046 Zuerich (CH); POLENSKE, Daniel, 3930 Visp (CH); GARMS, Stefan, 3902 Brig-Glis (CH); ESCHER, Christoph, 3912 Termen (CH); LORENZ, Andreas, 3922 Stalden (CH); BINER, Sonja, 3723 Kiental (CH); VON DER GRUEN, Mareile, 3932 Visperterminen (CH); KUEHBERGER, Herbert, 3904 Naters (CH); ZURBRIGGEN, Jonas, 3910 Saas-Grund (CH); IN-ALBON, Dieter, 3939 Eggerberg (CH); LUTZ, Beat, 3940 Steg-Hohtenn (CH)
(86) International application number: PCT/EP2016/077531
(87) International publication number: WO 2017/084995

(56) References cited:
- EP-A1- 1 767 528
- EP-A1- 1 990 336
- WO-A2-2008/149379

## Description

The invention discloses a method for the preparation of 1-methyl-3-(trifluoromethyl)-1H-pyrazol-5-ol with high selectivity with respect to the content of the isomer 1-methyl-5-(trifluoromethyl)-1H-pyrazol-3-ol.

### BACKGROUND OF THE INVENTION

The following abbreviations are used, if not otherwise stated:
ETFAA ethyl 4,4,4-trifluoroacetoacetate, compound of formula (3);
3-MTP 1-methyl-5-(trifluoromethyl)-1H-pyrazol-3-ol, compound of formula (2), the isomer; 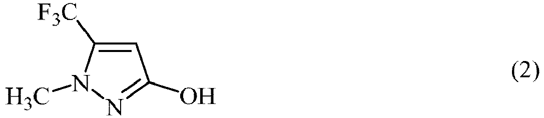
5-MTP 1-methyl-3-(trifluoromethyl)-1H-pyrazol-5-ol, compound of formula (1).
5-MTP is useful as an intermediate in the production of pharmaceutical and agricultural chemicals, such as herbicides such as pyroxasulfone.

Lee et al., J. Heterocyclic Chem. 1990, 27, 243-245, discloses a method for preparation of 5-MTP: Methyl hydrazine is added at room temperature to a mixture of ETFAA and water. After the reaction has subsided, the mixture is held at reflux for 2 h. The yield is 24.2 g (49%) of 5-MTP and 4.2 g (8%) of 3-MTP, selectivity of 6 : 1.

EP 1 767 528 A1 and EP1 990 336 A1 both disclose in an identical Reference Example 1 a method for preparation of 5-MTP: ETFAA is dissolved in 2 eq of acetic acid, at 10 °C aqueous methyl hydrazine is added over 1 h, then the solution is stirred for 1 h at room temperature and then for 5 h at 80 °C. Yield is 86.5%. Repetition of this Reference Example 1 showed a selectivity of 96 : 4 as described in the Comparative Example 1 of instant invention. There was a need for a method for preparation of 5-MTP which provides 5-MTP in high yield and in higher selectivity then known from the prior art and that does not require the use of acetic acid in stoichiometric or even higher amounts. Also better filtration and washing behavior was desired. Also the method should not require the addition of a solvent.

The prior art suggests to lower the temperature when mixing methyl hydrazine with the ETFAA for obtaining higher selectivity: Lee in 1990 uses room temperature and obtains a selectivity of 6 : 1, whereas Reference example 1 in EP 1 767 528 A1, with priority date 31.03.2004 and publication date 28.03.2007, and in EP1 990 336 A1, with priority date 14.02.2006 and publication date 12.11.2008, respectively uses 10 °C for the mixing, that is an even lower temperature compared to Lee at al., and obtains the higher selectivity of 96 : 4.

Unexpectedly, by mixing the ETFAA with the methyl hydrazine at elevated temperature, that is at a temperature well above room temperature, and in aqueous medium and in the presence of catalytic amounts of an acid, the selectivity can be enhanced while the yield is still high. When the ratio of the isomers is defined as the amount of the isomer with lower activation energy divided by the amount of the isomer with higher activation energy, it is surprising that raising of the temperature leads to a higher ratio of the isomers, that is to a higher selectivity, since in a reaction, that leads to two isomers, the isomer with the lower activation energy is favored at lower reaction temperature. Therefore to enhance the ratio of the isomers in such a case it is mandatory to lower the temperature. If the temperature is raised then the isomer with the higher activation energy is formed to a higher extent, thereby the ratio of the isomers is lowered. In this invention surprisingly it is vice versa.
5-MTP is obtained in form of large crystals, which allows for fast and effective filtration and washing, the use of acetic acid in stoichiometric or even higher amounts is not required, thereby the costs of the method are significantly lower compared to the method disclosed in Reference Example 1 of EP 1 767 528 A1 and of EP1 990 336 A1 respectively. The method provides the product in the form of larger crystals compared to prior art, furthermore the crystals have platelet like shape, where the prior art provides the crystals in form of needles. This improvement results in better filtration and washing behavior.

### SUMMARY OF THE INVENTION

Subject of the invention is a method for the preparation of compound of formula (1); 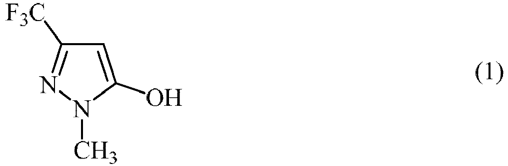 the method comprises a step ST1;
ST1 comprises a reaction REAC1 of compound of formula (3) 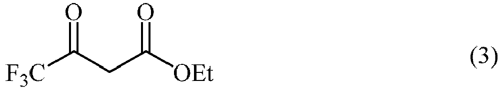 with methyl hydrazine;
in REAC1 compound of formula (3) and methyl hydrazine are brought into contact at a temperature TEMP1CONT and are reacted at a temperature TEMP1REAC;
TEMP1CONT and TEMP1REAC are from 50 to 140 °C;
methyl hydrazine is charged to compound of formula (3);
REAC1 is done in aqueous medium and without the addition of a solvent other than water or ethanol.

### DETAILED DESCRIPTION OF THE INVENTION

In another embodiment, subject of the invention is a method for the preparation of compound of formula (1); 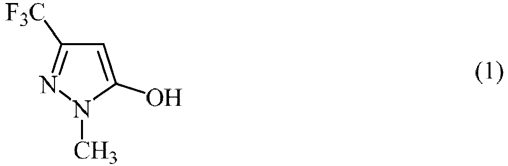 the method comprises a step ST1;
ST1 comprises a reaction REAC1 of compound of formula (3) 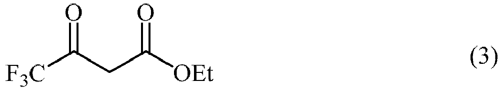 with methyl hydrazine;
in REAC1 compound of formula (3) and methyl hydrazine are brought into contact at a temperature TEMP1CONT and in the presence of an acid ACID1 and are reacted at a temperature TEMP1REAC and in the presence of ACID1;
TEMP1CONT and TEMP1REAC are from 50 to 140 °C;
ACID1 is selected from the group consisting of sulfuric acid, acetic acid, trifluoro acetic acid, H₃PO₄, methane sulfonic acid, formic acid, polymeric sulfonic acid resin, and mixtures thereof;
the molar amount of ACID1 is from 0.001 to 0.25 times of the molar amount of compound of formula (3);
REAC1 is done in aqueous medium and without the addition of a solvent other than water or ethanol.

Compound of formula (1), compound of formula (2) and compound of formula (3) can adopt various tautomeric forms, depending for instance on solvent or on pH, therefor their formulae comprise any respective tautomeric form.

Preferably, methyl hydrazine is charged to compound of formula (3).
Preferably, the charging of methyl hydrazine to compound of formula (3) is done in a time TIME1ADD, TIME1ADD is from 10 min to 6 h; preferably from 15 min to 4 h, more preferably from 20 min to 3 h, even more preferably from 25 min to 3 h.

Preferably, methyl hydrazine is used as an aqueous solution;
more preferably, methyl hydrazine is used as an aqueous solution of from 30 to 50 % (w/w); even more preferably of from 35 to 45 % (w/w).

Preferably, the molar amount of methyl hydrazine is from 0.9 to 1.5 times, more preferably from 0.95 to 1.25 times, even more preferably from 0.98 to 1.15 times, of the molar amount of compound of formula (3).
The aim of using methyl hydrazine in sub stoichiometric amounts is to avoid having residual methyl hydrazine, which is highly toxic, in the final product and in any waste streams.

Preferably, in REAC1 compound of formula (3) and methyl hydrazine are brought into contact in the presence of an acid ACID1 and are reacted in the presence of ACID1;
ACID1 is selected from the group consisting of sulfuric acid, acetic acid, trifluoro acetic acid, H₃PO₄, methane sulfonic acid, formic acid, polymeric sulfonic acid resin, and mixtures thereof;
more preferably, ACID1 is selected from the group consisting of sulfuric acid, trifluoro acetic acid, H₃PO₄, methane sulfonic acid, polymeric sulfonic acid resin, and mixtures thereof;
even more preferably, ACID1 is selected from the group consisting of sulfuric acid, trifluoro acetic acid, polymeric sulfonic acid resin, and mixtures thereof.

The polymeric sulfonic acid resin is preferably an acidic cation exchange resin, more preferably a strongly acidic cation exchange resin, for example such as used in heterogeneous acid catalysis.

Preferably, the polymeric sulfonic acid resin has an average molecular weight of from 1000 to 1000000 D; and/or
preferably, a concentration of acid sites of from 1 to 15, more preferably of from 1 to 11.6, even more preferably of from 1 to 10, especially of from 1 to 8, more especially of from 1 to 7 equivalents per kg resin; and/or
preferably, an acid number of from 1 to 650, more preferably of from 1 to 560, even more preferably of from 1 to 450, especially of from 1 to 350, more especially of from 50 to 650, even more especially of from 1 to 560, in particular of from 50 to 450, more in particular of from 50 to 350.

The concentration of acid sites is determined by the Master Test Method MTM 0232, Edition 1.4, ©Rohm and Haas Company, 1998, wherein the CATALYST VOLATILES are determined by the Master Test Method MTM 0126, Edition 1.6, ©Rohm and Haas Company, 2000.

The acid number is determined according to DIN EN ISO 3682. For further explanation of the acid number and for its relation to the concentration of acid sites see "BASF Handbuch Lackiertechnik", Artur Goldschmidt and Hans-Joachim Streitberger, Vincentz Verlag, 2002, ISBN 3-87870-324-4, chapter 2.3.2.2 (pages 272 to 273). According to the teaching therein, an concentration of acid sites of 1 equivalents per kg equals an acid number of 56, therefore a concentration of acid sites of 4.7 equivalents per kg equals an acid number of 263.

Especially, the polymeric sulfonic acid resin is selected from the group consisting of sulfonated polystyrene resins, sulfonated polystyrene resins cross linked with divinyl benzene and poly(2-acrylamido-2-methyl-1-propanesulfonic acid).

Sulfonated polystyrene resins cross linked with divinyl benzene are also called divinylbenzene-styrenesulfonic acid copolymer.

One example for a polymeric sulfonic acid resin is Amberlyst® 15 DRY.

Preferably, the molar amount of ACID1 is from 0.001 to 0.25 times, more preferably from 0.005 to 0.2 times, even more preferably from 0.005 to 0.15 times, especially from 0.005 to 0.125 times, more especially from 0.01 to 0.125 times, even more especially from 0.05 to 0.125 times, of the molar amount of compound of formula (3).
In another preferred embodiment, the molar amount of ACID1 is from 0.001 to 0.25 times, more preferably from 0.005 to 0.25 times, even more preferably from 0.01 to 0.25 times, especially from 0.01 to 0.2 times, more especially 0.05 to 0.2 times, even more especially from 0.05 to 0.15 times, in particular from 0.05 to 0.125 times, of the molar amount of compound of formula (3).

Preferably, acetic acid is not present in REAC1 in an amount over 1 eq, more preferably over 0.5, even more preferably over 0.25 eq, based on the molar amount of compound of formula (3);
more preferably, ACID1 is not present in REAC1 in an amount over 1 eq, more preferably over 0.5, even more preferably over 0.25 eq, based on the molar amount of compound of formula (3).

Preferably, TEMP1CONT and TEMP1REAC are from 60 to 120°C, more preferably from 70 to 120°C, even more preferably from 75 to 100°C, especially from 80 to 100 °C.
In another preferred embodiment, TEMP1CONT and TEMPI REAC are from 60 to 140°C, more preferably from 70 to 140°C, even more preferably from 75 to 140°C, especially from 80 to 140 °C.

Preferably, REAC1 is done at a pressure of from 0.1 to 10 bar, more preferably of from 0.1 to 5 bar, even more preferably of from 0.5 to 5 bar, especially of from 0.5 to 2.5 bar.
The pressure of REAC1 can be adjusted according to the chosen TEMP1CONT, to the chosen TEMP1REAC and to the boiling point of the reaction mixture that is formed when compound of formula (3) and methyl hydrazine are brought into contact.
Preferably, the reaction time TIME1REAC of REAC1 is from 0.5 h to 12 h, more preferably from 1 h to 6 h, even more preferably from 1 h to 4 h.

Preferably, ST1 can comprise a distillation DIST1 that is done during or after REAC1, wherein ethanol is distilled off;
more preferably, in DIST1 ethanol and water are distilled off.
Preferably the ethanol, that is distilled off, is the ethanol that is formed during REAC1.

Preferably, during or after DIST1 water is added to the reaction mixture.

Preferably, REAC 1 is done in aqueous medium.
Preferably, REAC 1 is done without the addition of a solvent other than water.
Preferably, REAC1 is done without the addition of a solvent other than water or ethanol.
More preferably, REAC1 is done in aqueous medium and without the addition of a solvent other than water.
More preferably, REAC1 is done in aqueous medium and without the addition of a solvent other than water or ethanol.
When REAC1 is done without the addition of a solvent other than water, then the only solvent other than water, that is present in REAC 1, is the ethanol that is formed during REAC 1.
When REAC1 is done without the addition of a solvent other than water or ethanol, then the only solvent other than water, that is present in REAC1, is the ethanol that is formed during REAC1 or that is added.
Preferably, when REAC1 is done in aqueous medium, then the methyl hydrazine is used as an aqueous solution;
more preferably, when REAC1 is done in aqueous medium and the methyl hydrazine is used as an aqueous solution, then the water that is present during REAC 1 is the water from the aqueous methyl hydrazin; that is no additional water is added.
When ethanol is added in or during REAC 1, the amount of ethanol, that is added, is preferably up to 10 times, more preferably up to 5 times, even more preferably up to 2 times, especially up to 1 times, either of the weight or of the molar amount of compound of formula (3).
Preferably, no ethanol is added.

ST1 can comprise the addition of a solvent SOLV1, SOLV1 is added after REAC1 or after DIST1, preferably SOLV1 is added after DIST1;
SOLV1 is selected from the group consisting of ethyl acetate, butyl acetate, valero nitrile, chloro benzene, dichloro benzene, 1,2-dichloro ethane, and mixtures thereof;
preferably, SOLV1 is selected from the group consisting of ethyl acetate, butyl acetate, valero nitrile, 1,2-dichloro ethane, and mixtures thereof;
more preferably, SOLV1 is selected from the group consisting of ethyl acetate, valero nitrile, 1,2-dichloro ethane, and mixtures thereof.

After ST1, compound of formula (1) can be isolated and purified by methods well-known to those skilled in the art. These include, for instance, cooling, filtration, washing after filtration and drying.
The product crystallizes after REAC1, during or after DIST1, or during or after a cooling.

Compound of formula (1), compound of formula (2) and compound of formula (3) are known compounds which are commercially available and/or can be produced according to known methods.

### Examples

In the examples the following applies, if not otherwise stated:
**Selectivity** is the ratio of compound of formula (1) : compound of formula (2). Selectivity is determined by NMR.

### HPLC:

Column: YMC-Pack ODS-A (250 mm x 4.6 mm x 5 micro meter), YMC Europe GmbH, 46539 Dinslaken, Germany
Gradient elution: Eluent A 0.1 % H3PO4 + 10 %v/v acetonitrile, Eluent B acetonitrile
At 40 °C: Start at 94.4 : 5.6 A : B; over 50 min to 33.3 : 66.7 A : B
UV detector at 230 nm
External Standard for HPLC: 5-MTP (Sigma-Aldrich, 97.0% determined by NMR)

### NMR referencing:

¹H-NMR: TMS delta 0 ppm

¹³C-NMR: DMSO-d₆ delta 39.51 ppm

¹⁹F-NMR: 1,4-Difluorobenzene delta -120.89 ppm

### Particle size distribution:

d_{xx,3} : xx = percentage of the sample, 3 = volume %

### Example 1 (with catalyst sulfuric acid)

Ethyl 4,4,4-trifluoroacetoacetate (100 g, 0.54 mol, 1 eq) and aqueous sulfuric acid 96% (w/w, 5.3 g, 0.05 mol, 0.09 eq) were placed in a 0.5 L double walled stirred glass reactor and heated to 85 °C. Aqueous methyl hydrazine 40% (w/w, 68.8 g, 0.60 mol, 1.11 eq) was added over a period of 30 min while the reaction temperature was maintained at 85 °C. Then the resulting reaction mixture was stirred for 2 h at 85 °C. Then distillate (42 g) was distilled off at ambient pressure and 95 °C during 1 h. During the distillation water (112 g) was added gradually. The resulting reaction mixture was cooled to 10 °C. Crystallized material was collected by filtration, washed with water (97 g), then dried under vacuum (50 mbar) and 60 °C yielding 78.9 g of compound of formula (1) (yield: 87.5%). The purity of the product was determined to be 98.5% w/w by ¹⁹F-NMR.

Selectivity was 99.2 : 0.8.

### Example 2 (with catalyst trifluoro acetic acid)

Example 1 was repeated with the sole difference that trifluoro acetic acid (0.1 eq) was used instead of the sulfuric acid (0.09 eq).

Yield was 83.9%.

Purity: 99.6% (w/w)

Selectivity was 97.2 :2.8.

### Comparative Example 1

Reference Example 1 of EP 1 767 528 A1 was repeated.

Yield was 85.8 %.

Selectivity was 95.2 : 4.8.

### Example 3 (without catalyst)

Ethyl 4,4,4-trifluoroacetoacetate (150 g, 0.81 mol, 1 eq) was heated to 85 °C. Aqueous methyl hydrazine 40% (w/w, 103.2 g, 0.9 mol, 1.10 eq) was added over a period of 2 h while the reaction temperature was maintained at 90 to 94 °C. Then the reaction mixture was stirred for 2 h at 90 to 94 °C. Water (260 g) was added. Then distillate (47 g) was distilled off at ambient pressure and at 92 to 96 °C during 40 min. The resulting reaction mixture was cooled to 10 °C. Crystallized material was collected by filtration, washed with water (230 g), then dried under vacuum (20 mbar) and 50 °C yielding 97.4 g of compound of formula (1) (yield: 72.4%). The purity of the product was determined to be 99.7% w/w by ¹H-NMR. Selectivity was 98.1 : 1.9.

## Claims

1. Method for the preparation of compound of formula (1); 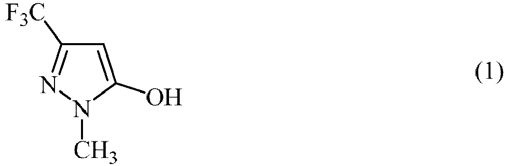 the method comprises a step ST1;
ST1 comprises a reaction REAC1 of compound of formula (3) 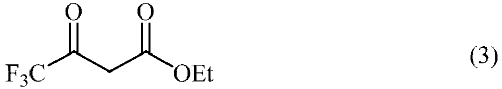 with methyl hydrazine;
in REAC1 compound of formula (3) and methyl hydrazine are brought into contact at a temperature TEMP1CONT and are reacted at a temperature TEMP1REAC;
TEMP1CONT and TEMP1REAC are from 50 to 140 °C;
methyl hydrazine is charged to compound of formula (3);
REAC1 is done in aqueous medium and without the addition of a solvent other than water or ethanol.

2. Method according to claim 1, wherein
the charging of methyl hydrazine to compound of formula (3) is done in a time TIME1ADD, TIME1ADD is from 10 min to 6 h.

3. Method according to claim 1 or 2, wherein
REAC1 is done in aqueous medium and without the addition of a solvent other than water.

4. Method according to one or more of claim 1 to 3, wherein
in REAC1 compound of formula (3) and methyl hydrazine are brought into contact in the presence of an acid ACID1 and are reacted in the presence of ACID1;
ACID1 is selected from the group consisting of sulfuric acid, acetic acid, trifluoro acetic acid, H₃PO₄, methane sulfonic acid, formic acid, polymeric sulfonic acid resin, and mixtures thereof.

5. Method according to claim 4, wherein
the molar amount of ACID1 is from 0.001 to 0.25 times of the molar amount of compound of formula (3).

6. Method according to claim 4 or 5, wherein
the molar amount of ACID1 is from 0.005 to 0.25 times of the molar amount of compound of formula (3).

7. Method according to one or more of claims 1 to 6, wherein
methyl hydrazine is used as an aqueous solution.

8. Method according to one or more of claims 1 to 7, wherein
TEMP1CONT and TEMP1REAC are from 60 to 140°C.

9. Method according to one or more of claims 1 to 8, wherein
ST1 comprises a distillation DIST1 that is done during or after REAC1, wherein ethanol is distilled off.

10. Method according to one or more of claims 1 to 9, wherein
ST1 comprises the addition of a solvent SOLV1, SOLV1 is added after REAC1;
SOLV1 is selected from the group consisting of ethyl acetate, butyl acetate, valero nitrile, chloro benzene, dichloro benzene, 1,2-dichloro ethane, and mixtures thereof.

## Patentansprüche

1. Verfahren zur Herstellung der Verbindung der Formel (1); 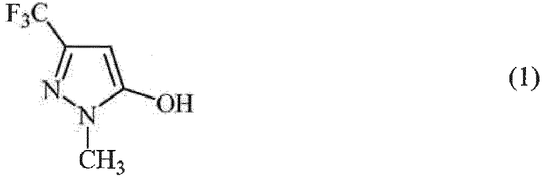 wobei das Verfahren einen Schritt SCHI umfasst;
SCHI eine Umsetzung UMS1 der Verbindung der Formel (3) 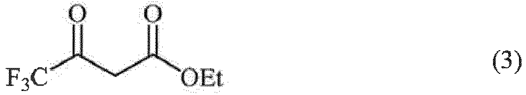 mit Methylhydrazin umfasst;
in UMS1 die Verbindung der Formel (3) und Methylhydrazin bei einer Temperatur TEMP1KONT in Kontakt gebracht und bei einer Temperatur TEMP1UMS umgesetzt werden;
TEMP1KONT und TEMP1UMS 50 bis 140 °C betragen; Methylhydrazin zu der Verbindung der Formel (3) gegeben wird;
UMS1 in wässrigem Medium und ohne Zugabe eines anderen Lösungsmittels als Wasser oder Ethanol durchgeführt wird.

2. Verfahren nach Anspruch 1, bei dem die Zugabe von Methylhydrazin zu der Verbindung der Formel (3) in einer Zeit ZEIT1ZUG erfolgt, ZEIT1ZUG 10 min bis 6 h beträgt.

3. Verfahren nach Anspruch 1 oder 2, bei dem UMS1 in wässrigem Medium und ohne Zugabe eines anderen Lösungsmittels als Wasser erfolgt.

4. Verfahren nach einem oder mehreren der Ansprüche 1 bis 3, bei dem
in UMS1 die Verbindung der Formel (3) und Methylhydrazin in Gegenwart einer Säure SÄURE1 in Kontakt gebracht und in Gegenwart von SÄURE1 umgesetzt werden;
SÄURE1 aus der Gruppe bestehend aus Schwefelsäure, Essigsäure, Trifluoressigsäure, H₃PO₄, Methansulfonsäure, Ameisensäure, polymerem Sulfonsäureharz und Mischungen davon ausgewählt wird.

5. Verfahren nach Anspruch 4, bei dem die molare Menge von SÄURE1 das 0,001- bis 0,25-fache der molaren Menge der Verbindung der Formel (3) beträgt.

6. Verfahren nach Anspruch 4 oder 5, bei dem die molare Menge von SÄURE1 das 0,005- bis 0,25-fache der molaren Menge der Verbindung der Formel (3) beträgt.

7. Verfahren nach einem oder mehreren der Ansprüche 1 bis 6, bei dem
Methylhydrazin in Form einer wässrigen Lösung verwendet wird.

8. Verfahren nach einem oder mehreren der Ansprüche 1 bis 7, bei dem
TEMP1KONT und TEMP1UMS 60 bis 140 °C betragen.

9. Verfahren nach einem oder mehreren der Ansprüche 1 bis 8, bei dem
SCHI eine Destillation DEST1 umfasst, die während oder nach UMS1 durchgeführt wird, wobei Ethanol abdestilliert wird.

10. Verfahren nach einem oder mehreren der Ansprüche 1 bis 9, bei dem
SCHI die Zugabe eines Lösungsmittels LÖS1 umfasst, LÖS1 nach UMS1 zugegeben wird; LÖS1 aus der Gruppe bestehend aus Essigsäureethylester, Essigsäurebutylester, Valeronitril, Chlorbenzol, Dichlorbenzol, 1,2-Dichlorethan und Mischungen davon ausgewählt wird.

## Revendications

1. Procédé de synthèse du composé de formule (1) ; 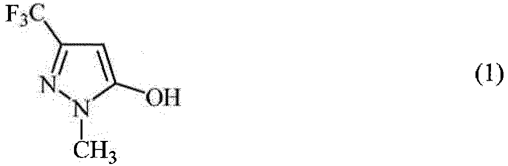 le procédé comprend une étape ST1 ;
ST1 comprend une réaction REAC1 du composé de formule (3) 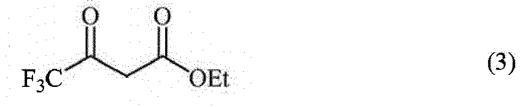 avec la méthylhydrazine ;
dans REAC1, le composé de formule (3) et la méthylhydrazine sont mis en contact à une température TEMP1CONT et réagissent à une température TEMP1REAC ;
TEMP1CONT et TEMP1REAC sont comprises entre 50 et 140 °C ;
la méthylhydrazine est chargée dans le composé de formule (3) ;
REAC1 est mise en oeuvre dans un milieu aqueux et sans l'ajout d'un solvant différent de l'eau ou de l'éthanol.

2. Procédé selon la revendication 1, où le chargement de la méthylhydrazine sur le composé de formule (3) est mis en oeuvre à un temps TIME1ADD, TIME1ADD est comprise entre 10 min et 6 h.

3. Procédé selon la revendication 1 ou 2, où REAC1 est mise en oeuvre dans un milieu aqueux et sans l'ajout d'un solvant différent de l'eau.

4. Procédé selon une ou plusieurs des revendications 1 à 3, où
dans REAC1, le composé de formule (3) et la méthylhydrazine sont mis en contact en présence d'un acide ACID1 et réagissent en présence d'ACID1 ;
ACID1 est choisi dans le groupe constitué par les suivants : acide sulfurique, acide acétique, acide trifluoroacétique, H₃PO₄, acide méthanesulfonique, acide formique, résine d'acide sulfonique polymère, et leurs mélanges.

5. Procédé selon la revendication 4, où la quantité molaire d'ACID1 est comprise entre 0,001 et 0,25 fois la quantité molaire du composé de formule (3) .

6. Procédé selon la revendication 4 ou 5, où la quantité molaire d'ACID1 est comprise entre 0,005 et 0,25 fois la quantité molaire du composé de formule (3).

7. Procédé selon une ou plusieurs des revendications 1 à 6, où
la méthylhydrazine est utilisée sous forme de solution aqueuse.

8. Procédé selon une ou plusieurs des revendications 1 à 7, où
TEMP1CONT et TEMP1REAC sont comprises entre 60 et 140 °C.

9. Procédé selon une ou plusieurs des revendications 1 à 8, où
ST1 comprend une distillation DIST1 qui est mise en oeuvre avant ou après REAC1, où l'éthanol est éliminé par distillation.

10. Procédé selon une ou plusieurs des revendications 1 à 9, où
ST1 comprend l'addition d'un solvant SOLV1, SOLV1 est ajouté après REAC1 ;
SOLV1 est choisi dans le groupe constitué par les suivants : acétate d'éthyle, acétate de butyle, valéronitrile, chlorobenzène, dichlorobenzène, 1,2-dichloroéthane, et leurs mélanges.
